# EUROPEAN PATENT APPLICATION

(11) **EP 2 422 870 A1**
(43) Date of publication of application: **29.02.2012**
(21) Application number: 10177231.7
(22) Date of filing: 16.09.2010
(51) Int. Cl.: B01D 53/84, C12M 1/00

(54) **Extraction of co2 gas**

(30) Priority: 26.08.2010 US 969398; 26.08.2010 US 869376
(71) Applicant: SFN Biosystems, Inc., Calgary, AB T2G 3ZB (CA)
(72) Inventor: Koch, Edward John, Calgary Alberta T2J 2C1 (CA); Copeland, Madison, Calgary Alberta T2J 2C1 (CA); Audet, Adrian David, Calgary Alberta T3E 1Y7 (CA)
(74) Representative: Dempster, Benjamin John Naftel

(57) **Abstract**

A photo-bioreactor (17) is used for extraction of carbon dioxide from exhaust gases of an engine (10) used for compression of natural gas by providing at least a vessel (18) and in each contacting the gases with a liquid medium containing photo-synthetic organisms. The vessel (18) receives the gases and is controlled to manage the temperature and dwell time to take into account the reducing CO2 content. The gases (23) are introduced using directional diffusers at the bottom of the bath which generate a flow in the water. The vessel (18) has a water supply (222) and harvesting system (34) so that the excess organisms grown are extracted from each for harvesting (37). A gas recirculation system (46) is controlled to manage the gas dwell time in the vessel (18). The divider walls contain the electrically powered lights (28) between two transparent sheets.

## Description

This invention relates to a system to extract CO2 from gas. Particularly but not exclusively, the system is targeted at stationary engines burning natural gas commonly used for gas compression but the system could apply to any combustion of fossil fuels.

### BACKGROUND OF THE INVENTION

The following patents have been located which are relevant in this field:
W020088008262 (Lewnard) published 17th January 2008 by Greenfuels shows a CO2 extraction system for exhaust using photo synthetic organisms which are grown in a tank illuminated by light from above and are harvested to produce fuel products.
WO02007/011343 (Berzin) published 25th January 2007 by Greenfuels shows similar system.
WO2007/134141 (Bayless) published 22nd November 2007 by OHIO University provides a similar system which uses optical fibers to carry the ambient light to the medium.
US Patent 6,667,171 (Bayless) issued December 23rd 2003 by OHIO University relates generally to sequestration of CO2 by microbes attached to surfaces in a containment chamber.
US Patent 5,104,803 (Delente) issued April 14th 1992 by Martek shows a single reactor cell with side by side banks of light tubes.
US Patent 6,602,703 (Dutil) issued August 5th 2003 by CO2 Solutions shows a similar cell using florescent tubes with a cleaning device.
US Patent 6,287,852 (Kondo) issued September 11th 2001 by Matsushita which shows an arrangement of this type using parallel cells with light transfer ducts between the cells.
US Patent 5,659,977 (Jensen) issued August 26th 1997 by Cyanotech which provides a generating plant using a bioreactor
US Patent 6,083,740 (Kodo) issued July 4th 2000 by Spirolina shows a bioreactor defined by an array of tubular cells in parallel.
US Patent 5,741,702 (Lorenz) issued April 21st 1998 shows an arrangement for transferring natural light into a bioreactor.
US patent 5,804,432 (Knapp) issued September 8th 1998 shows a bioreactor defined by a plurality of vessels in series. The bioreactor is designed to use bacteria with oxygen to remove volatile organic contaminants (VOCs) from petroleum contaminated water as. No light is required in the bacteria bioreactor. Flow of the liquid medium between the vessels of the bioreactor is accomplished by gravity with each container overflowing into the next one.
US Patent Application 2003/0059932 (Craigie) published March 27th 2003 by National Research Counsel of Canada which relates to the servicing of light supply tubes for a bioreactor.

### SUMMARY OF THE INVENTION

It is one object of the invention to provide a system to extract CO2 gas from combustion.

According to a first independent aspect of the invention there is provided a method for extraction of carbon dioxide from exhaust gases from combustion of a fossil fuel, the method comprising:
providing a supply of exhaust gas from combustion of a fossil fuel;
extracting heat using a heat exchanger to cool the exhaust gas and to generate a supply of a heated liquid;
providing a vessel containing a liquid medium carrying photo-synthetic organisms;
the vessel having a plurality of upstanding walls;
providing light energy to the liquid medium;
supplying the cooled exhaust gas to the liquid medium in the vessel;
extracting the liquid medium containing the organisms from the vessel, harvesting at least some of the organisms from the extracted liquid medium and returning at least some of the liquid medium after the extraction to the vessel;
and heating the vessel using the heated liquid by passing the liquid through at least one heating duct mounted in contact within with at least one wall of the vessel so as to apply heat to the wall.

Preferably the duct is carried on an outside surface of the wall.

Preferably the inside surface of the wall is flush for cleaning.

Preferably the cooled exhaust gas is supplied to the vessel though a plurality of diffusers at the base of the vessel supplied through a plurality of communication conduits passing through the medium in the vessel and wherein the communication conduits are arranged to act as heat exchanger transferring heat from the conduits to the medium in the vessel.

Preferably the heat supplied by the cooled exhaust gas and by the heating liquid is controlled to maintain the temperature of the medium in the vessel at a temperature for active growth of the organisms.

Preferably the gas is supplied to the vessel by a supply construction defined by a horizontal array of supply pipes extending across the base of the vessel and plurality of diffusers mounted on the array above the array, each diffuser including a ceramic nozzle for generating bubbles in the medium.

Preferably the gas is supplied to the vessel by a supply construction extending across the base of the vessel and wherein the liquid medium is extracted by a suction system extending across the base underneath the supply construction.

Preferably there is provided a sump in the base.

According to a second independent aspect of the invention there is provided a method for extraction of carbon dioxide from exhaust gases from combustion of a fossil fuel, the method comprising:
providing a supply of exhaust gas from combustion of a fossil fuel;
extracting heat using a heat exchanger to cool the exhaust gas and to generate a supply of a heated liquid;
providing a vessel containing a liquid medium carrying photo-synthetic organisms;
the vessel having a plurality of upstanding walls;
providing light energy to the liquid medium;
supplying the cooled exhaust gas to the liquid medium in the vessel;
extracting the liquid medium containing the organisms from the vessel, harvesting at least some of the organisms from the extracted liquid medium and returning at least some of the liquid medium after the extraction to the vessel;
and heating the medium using the heated liquid ;
   wherein the cooled exhaust gas is supplied to the vessel though a plurality of diffusers at the base of the vessel supplied through a plurality of communication conduits passing through the medium in the vessel and wherein the communication conduits are arranged to act as a heat exchanger transferring heat from the conduits to the medium in the vessel.

Preferably the gas is supplied to the vessel by a supply construction defined by a horizontal array of supply pipes extending across the base of the vessel and plurality of diffusers mounted on the array above the array, each diffuser including a ceramic nozzle for generating bubbles in the medium.

Preferably the gas is supplied to the vessel by a supply construction extending across the base of the vessel and wherein the liquid medium is extracted by a suction system extending across the base underneath the supply construction.

Preferably there is provided a sump in the base.

According to a third independent aspect of the invention there is provided a method for extraction of carbon dioxide from exhaust gases from combustion of a fossil fuel, the method comprising:
providing a supply of exhaust gas from combustion of a fossil fuel;
extracting heat using a heat exchanger to cool the exhaust gas and to generate a supply of a heated liquid;
extracting water vapor from the exhaust gas to form a supply of water;
providing a vessel containing a liquid medium including water carrying photo-synthetic organisms;
providing light energy to the liquid medium;
supplying the cooled exhaust gas to the liquid medium in the vessel;
extracting the liquid medium containing the organisms from the vessel, harvesting at least some of the organisms from the extracted liquid medium and returning some of the liquid medium including some of the water after the extraction to the vessel;
and adding at least some of the supply of water to the vessel to provide a make-up supply of water to replace water removed during the extracting of the organisms.

Preferably the amount of water extracted from the exhaust gas is greater than the amount of water removed during the extracting of the organisms.

Preferably there is provided a heat transfer system arranged to use heat from the heat exchanger to dewater the liquid medium from the liquid medium during the extracting of the organisms.

Preferably the vessel has a respective liquid medium extraction system arranged to continuously extract the liquid medium containing the organisms from said the vessel and a harvesting system arranged to extract some of the organisms from the extracted liquid medium so as to provide a stream of harvested organisms in a liquid medium and a stream of liquid medium to be returned to the vessel.

Preferably there is provided a gas recirculation system for withdrawing gas from the vessel and returning the gas to the gas supply system to the liquid medium in the vessel

Preferably there is provided a heat transfer system arranged to use heat from the heat exchanger to dewater the liquid medium from the liquid medium extraction system.

Preferably the engine is an engine fueled by natural gas and used for natural gas compression.

According to a further independent aspect of the invention there is provided a method for extraction of carbon dioxide from a gas, comprising:
providing a vessel containing a liquid medium carrying photo-synthetic organisms;
providing light energy to the liquid medium;
supplying the gas to the liquid medium in the vessel;
extracting the liquid medium containing the organisms from the vessel, harvesting at least some of the organisms from the extracted liquid medium and returning at least some of the liquid medium after the extraction to the vessel;
wherein the light energy is supplied to the vessel so as to generate a lighted area in the vessel in which the medium is supplied with light energy and to generate a dark area in the vessel in which the medium is remote from the light energy;
and generating light and dark cycles for the organisms by circulating the medium between the light and dark areas.

Preferably the light and dark cycles generated are arranged to provide natural light and dark cycles necessary for proper growth of the organisms.

Preferably the lighted area forms a proportion of the total area which is in the range 50% to 80%.

Preferably the rate of circulation is varied to maximize the growth rate of the organisms.

Preferably the lighted and dark areas are open to one another within the vessel and the circulation is caused by free currents within the vessel.

Preferably the circulation is effected without a pathway which defines a path of the medium.

Preferably the circulation is effected by circulation fans within the dark area of the vessel.

Preferably the lighted area is defined by a section of the vessel containing banks of light sources and the dark area contains none of the light sources.

Preferably the light sources in the lighted area are arranged at spacing to provide light to substantially all of the organisms in the lighted area.

Preferably an interface between the lighted and dark areas is formed by an end face of the banks of light sources without any impediment to flow therebetween.

Preferably there are a plurality of banks as wherein there are provided circulation fans between the banks.

According to a further independent aspect of the invention there is provided a method for extraction of carbon dioxide from a gas, comprising:
providing a vessel containing a liquid medium carrying photo-synthetic organisms;
the vessel having a base, upstanding side walls and a top;
providing light energy to the liquid medium;
supplying the gas to the liquid medium in the vessel;
extracting the liquid medium containing the organisms from the vessel, harvesting at least some of the organisms from the extracted liquid medium and returning at least some of the liquid medium after the extraction to the vessel;
wherein the light energy is supplied by a plurality of banks of light sources;
wherein the gas is supplied to the vessel by a supply construction defined by a plurality of diffusers at a base of the vessel and supply pipes extending across the base;
removing the banks of light sources from the vessel through the top;
removing the supply construction;
when the light sources and the supply construction are removed the upstanding side walls are flush with no outstanding components;
and cleaning the upstanding flush side walls.

Preferably the banks of light sources are suspended into the tank from supports at the top.

Preferably the banks of lights each comprise a plurality of parallel spaced florescent tubes carried on a common mounting assembly.

Preferably each tube is surrounded by a protecting sleeve.

Preferably the tubes of each bank are horizontal and the banks are vertical and arranged side by side.

Preferably the gas is supplied to the vessel by a supply construction defined by a horizontal array of supply pipes extending across the base of the vessel and plurality of diffusers mounted on the array above the array, each diffuser including a ceramic nozzle for generating bubbles in the medium.

Preferably the gas is supplied to the vessel by a supply construction extending across the base of the vessel and wherein the liquid medium is extracted by a suction system extending across the base underneath the supply construction.

Preferably there is provided a sump in the base.

According to a further independent aspect of the invention there is provided a method for extraction of carbon dioxide from a gas, comprising:
providing a vessel containing a liquid medium carrying photo-synthetic organisms;
providing light energy to the liquid medium;
supplying the gas to the liquid medium in the vessel;
extracting the liquid medium containing the organisms from the vessel, harvesting at least some of the organisms from the extracted liquid medium and returning at least some of the liquid medium after the extraction to the vessel;
wherein the density of the organisms in the medium is greater than greater than 0.5 grams per liter of liquid medium (0.075 ounces per US Gallon of liquid medium)..

Preferably the level of carbon dioxide introduced into the medium is greater than .062 x 10-7 m3/sec per liter of liquid medium (0.0008517 CFM per gallon of liquid medium).

Preferably there is provided a gas recirculation system for withdrawing gas from the vessel and returning the gas to the gas supply system to the liquid medium in said at least one vessel and wherein the recirculation system is arranged so that the gas has a retention time of greater than 0.5 seconds per foot of liquid medium depth (1.64 seconds per meter of liquid medium depth).

Preferably the level of light energy introduced into the medium is greater than 0.045 watts per liter (or 0.170 watts per US gallon).

Preferably the rate of extraction is greater than 1.5 grams per liter of liquid medium per day (0.20 ounces per US Gallon of liquid medium per day).

In general the apparatus described in more detail hereinafter arranged for extraction of carbon dioxide from exhaust gases from an engine burning fossil fuel, comprises:
a duct arranged to receive the exhaust gases;
a heat exchanger arranged to extract heat from the exhaust gases;
a photo-bioreactor arranged to receive the exhaust gases and to contact the gases with a liquid medium containing photo-synthetic organisms;
the bioreactor including:
   at least one vessel containing the liquid medium;
   a plurality of banks of electrically powered lights in said at least one vessel arranged to supply illumination to the liquid medium;
   a guide wall system in said at least one vessel arranged to form a path for the liquid medium;
   a gas supply system arranged to continuously supply the gas from the duct to the liquid medium in said at least one vessel;
   a liquid medium system arranged to continuously supply the liquid medium to said at least one vessel;
   a liquid medium extraction system arranged to continuously extract the liquid medium containing the organisms from said at least one vessel; and
   a gas extraction system arranged to continuously extract the gas from said at least one vessel;
   a gas discharge arranged to discharge the gas from the gas extraction system to atmosphere;
   a harvesting system arranged to extract some of the organisms from the extracted liquid medium so as to provide a stream of harvested organisms in a liquid medium and a stream of liquid medium to be returned to the bioreactor;
   a conversion system arranged to take the harvested organisms and to convert the harvested organisms to a useable fuel product;
   a return system arranged to return the stream of liquid medium to the bioreactor;
   an input for adding nutrients to the liquid medium;
   an input for adding organisms to the liquid medium;
   a heating system and/or cooling system arranged to take heat from the heat exchanger and to apply the heat to the bioreactor to maintain a temperature in the bioreactor within a predetermine range for growing the organisms.

Preferably the bioreactor includes a plurality of vessels.

Preferably the vessels are arranged in series such that each is supplied with gas taken from a previous vessel of the series and preferably each vessel has a respective liquid medium extraction system arranged to continuously extract the liquid medium containing the organisms from said the vessel and a harvesting system arranged to extract some of the organisms from the extracted liquid medium so as to provide a stream of harvested organisms in a liquid medium and a stream of liquid medium to be returned to the vessel.

Preferably the flow rate of liquid medium is controlled in each vessel so that a dwell time of the liquid medium in a vessel is longer for later vessels of the series than a first one of the vessels of the series as the amount of CO2 is decreased.

Preferably each vessel has a respective heating system controlled to maintain the temperature of that vessel independently of the other vessels of the series.

Preferably there is provided a gas recirculation system for withdrawing gas from the vessel and returning the gas to the gas supply system to the liquid medium in the vessel

Preferably each vessel of the series has a respective gas recirculation system for withdrawing gas from the vessel and returning the gas to the gas supply system to the liquid medium in the vessel

Preferably there is provided a sensor for measuring CO2 content in the gas as supplied to the vessel and as discharged from the vessel which is used to control the volume of gas recirculation.

Preferably the defines a bath with the guide walls defining a flow path of the liquid medium through the bath and the gas recirculation system includes an extraction duct system arranged to take recirculation from overhead from the bath at spaced positions along the path.

Preferably the vessel defines a bath with the guide walls forming dividers in the vessel defining a labyrinth flow path of the liquid medium through the bath.

Preferably the dividers are formed of two parallel transparent sheets with the lights between, where the lights preferably are vertical florescent light tubes through the height of the bath.

Preferably the gas supply system comprises a plurality of diffusers at the bottom of the vessel, where the diffusers are preferably arranged to be directional along the vessel so as to cause flow of liquid medium along the vessel.

Preferably the electrically heated lights use electricity taken from an external electric supply rather than from electricity generated from the waste energy of the engine output.

Preferably the vessel and the heat exchanger are arranged such that a heat supply acts to maintain a required temperature in the bioreactor on the coldest days without introduction of extra heat and on remaining days excess heat is discarded. In this way no additional heat is required. In this way cooling can preferably be avoided.

Preferably there is provided a blower at the duct to ensure that no back pressure is applied into the duct to the engine.

Preferably there is provided a bypass valve in the duct arranged to release the exhaust gases to atmosphere in the event that a process interruption causes back pressure to develop to the engine.

Preferably the heating system is arranged to transfer heat from a first of the vessels of the series to others of the series.

Preferably there is provided a heat transfer system arranged to use heat from the heat exchanger to dewater the liquid medium from the liquid medium extraction system.

In particular the engine is preferably an engine fueled by natural gas and used for natural gas compression.

The reduction of CO2 content is achieved by taking the exhaust from the engine and circulating it through a bioreactor. The bioreactor consists of a series of vessels which sequentially reduce the CO2 content of the exhaust. The bioreactor contains algae, which convert the CO2 into oxygen in the presence of nutrients and light. In the process of converting the CO2 into oxygen, the algae will multiply. The algae are harvested and processed into biodiesel and associated by-products.

The exhaust created by internal combustion engine is cooled and the associated heat can be recovered (heat exchangers) for use in the process. The heat extracted can optionally be used for heating, or electrical generation using an organic Rankin cycle generation system.

The exhaust is introduced into the bioreactor. Nutrients and algae are mixed and introduced into the bioreactor. Light is introduced into the bioreactor. The algae utilize the light, CO2, and nutrients to produce oxygen and to reproduce whereupon algae is removed from the bioreactor, separated from the circulation water, and processed into biodiesel and associated products. The water and remaining algae are mixed with nutrients and used again. The exhaust is released to the atmosphere after being cleaned of CO2.

The bioreactor is formed by a series of vessels. These vessels are maintained at the correct temperature for algae growth. The temperature is controlled by an automated system, which uses heat from the engine exhaust and optionally cooling from coolers as required. The process operates continuously.

Each vessel contains a lighting system which provides intensive lighting for the growth of algae using florescent or LED light.

Each vessel contains an algae/nutrient mix circulating system. This system allows for the introduction of the algae/nutrient mix and for the removal of the algae/nutrient mix. Circulation of the algae/nutrient mix inside the vessel can be achieved by the use of oriented exhaust gas circulation jets. The movement of the exhaust gas through the nozzle will cause the agitation and movement of the algae/nutrient mix in the vessel.

Each vessel contains an exhaust gas circulation system. This system allows for the introduction of exhaust gas and the removal of exhaust gas (gas flow through the vessel at the equivalent mass flow rate of the exhaust leaving the engine). It also has a related system that allows for exhaust gas in the vessel to be re-circulated through the algae / nutrient mix. The exhaust is collected at the top of the vessel and re-injected into the exhaust feed line. This recirculation of exhaust gas is controlled to result in optimal algae growing conditions and CO2 removal in each vessel. The exhaust gas is circulated through the directional nozzles where the orientation of the nozzles controls the flow of algae/nutrient mix through the vessel

The exhaust travels through the vessels in series with the CO2 content of the exhaust decreased in each tank. The residence time of the algae/nutrient mix in each vessel is thus different and is controlled to optimize the CO2 removal and algae growth.

### BRIEF DESCRIPTION OF THE DRAWINGS

One embodiment of the invention will now be described in conjunction with the accompanying drawings in which:
Figure 1 is a schematic illustration of an apparatus according to the present invention including a bioreactor divided into a plurality of separate vessels.
Figure 2 is a horizontal cross section of one vessel of the bioreactor.
Figure 3 is a cross section along the lines 3-3 of the vessel of Figure 2.
Figure 4 is a cross section along the lines 4-4 of the vessel of Figure 2.
Figure 5 is a cross section along the lines 5-5 of the vessel of Figure 2.
Figure 6 is a schematic illustration of a second embodiment of apparatus according to the present invention including a single bioreactor vessel.
Figure 7 is a vertical longitudinal cross section of the bioreactor vessel of Figure 6.
Figure 8 is a vertical transverse cross section of the bioreactor vessel of Figure 6.
Figure 9 is a vertical transverse cross section of the bioreactor vessel of Figure 6 showing the heating element at one part of the side wall.
Figure 10 is an isometric partly exploded view of the bioreactor vessel of Figure 6 showing lighting banks.

In the drawings like characters of reference indicate corresponding parts in the different figures.

### DETAILED DESCRIPTION

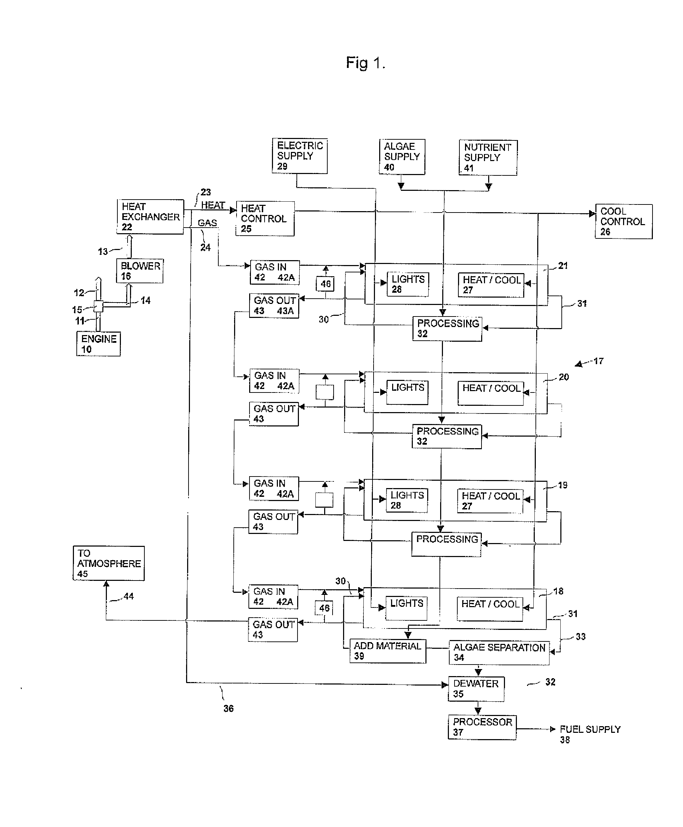

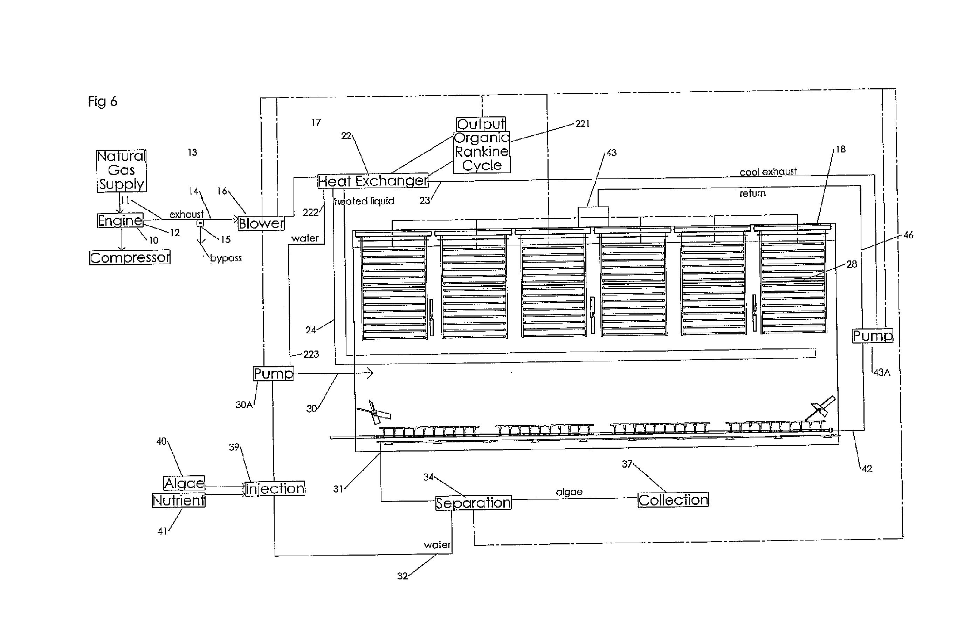

The apparatus shown schematically in Figure 1 and 6 includes an engine 10 which generates exhaust through a discharge duct 11 for supplying the exhaust to atmosphere through an outlet 12.

The engine concerned is primarily a stationary engine of the type used for compressing natural gas where the engine is often located in the remote location and uses as a supply fuel a stream of the natural gas itself which is intended to be compressed.

There are many engines of this type in wide use in gas producing regions and particularly in Canada where such engines utilize a significant portion of the natural gas and produce carbon dioxide emissions which are as much as 30% of the total emissions generated by all sources in Canada. The engines are commonly located in remote cold locations where the exterior temperature is often below 0° during the winter.

In some cases the exhaust gases in the duct 11 are supplemented by a carbon dioxide stream from the carbon dioxide extracted from the natural gas stream using conventional processes such as amine extraction.

The apparatus for extracting the carbon dioxide is generally indicated at 13 and includes an inlet duct 14 for taking the exhaust gases from the duct 11 and supplying them into the apparatus. A valve 15 is provided which closes off the duct 11 and allows all of the exhaust gases to be drawn into the apparatus described herein.

It is important for operation of the engine that there be no back pressure from the apparatus so that the engine operates in its normal condition without any effect on the exhaust discharge which can affect engine function. For this reason there is provided the valve 15 which acts as a bypass so as to shut off the apparatus and return the exhaust to the discharge 12 in the event that any process interruption occurs in the apparatus which would lead to a back pressure against the engine 10.

The duct 14 is connected to a blower or fan 16 which provides all flow and pressure for the operation of the apparatus and thus avoids the back pressure on the engine 10. The blower can be controlled so as to maintain the pressure at the duct 11 at the required level for normal engine operation.

The apparatus comprises a bioreactor generally indicated at 17 which is formed from a plurality of separate bio reactor vessels indicated at 18, 19, 20 and 21 respectively in Figure 1. In Figure 6 only a single vessel 18 is shown but this can be one of a plurality or can be a single vessel depending on dimensions and requirements. The number shown in the apparatus present is for such vessels but of course it will be appreciated that the number of vessels can vary in dependence upon the requirements for the process. The vessels can be used in series as shown in Figure 1 or can be used in parallel so that the cooled exhaust gas stream is split into separate pars to be fed to separate ones of the vessels.

In general the bioreactor utilizes a photo-synthetic organism such as algae. Such organisms are well known and commercially available and can be selected for use in generating various output materials such as fuels after the algae are harvested. In the present example it is intended that the algae be selected for production of bio-diesel since such algae are readily available and are of a type which can be readily managed in a bio reactor of the type described hereinafter.

The apparatus further includes a heat exchanger 22 which receives the exhaust from the blower 16 and produces from that exhaust a stream of the gas indicated at 23 and a heat stream provided on a supply duct 24. The heat stream may be carried by any fluid so that the heat can be used in various parts of the process. In particular the heat stream is utilized for supplying heat to each of the vessels 18, 19, 20 and 21 so as to maintain each vessel at a required temperature for growing the organisms within the liquid within the vessel. For this purpose, in Figure 1, a heat control system is schematically indicated at 25 which controls the supply of the heat to the individual vessels bearing in mind that the vessels will have different heat requirements due to the different processing conditions within each of the vessels. The heat control system therefore supplies the heated fluid to each of the vessels through a separate supply line and suitable monitoring systems are provided within the vessels to ensure the required temperature is maintained and the necessary control signals are transmitted back to the control system 25.

In addition it is in some cases possible and in some cases necessary to transfer heat from a first one of the vessels indicated at 21 through a last one of the vessels indicated at 18 or to one of the other vessels since in some situations one or more of the vessels maybe insufficiently heated and one or more vessels may be in a situation which provides available heat, bearing in mind the different operating characteristics within the individual vessels. Thus the control system 25 may also be arranged to control the supply of heat to the individual vessel so as to act to transfer heat from one vessel to another.

In some cases cooling is also necessary so that a cooling system 26 can be provided as an optional element which provides cooling fluid to the individual vessels again controlled by the control system 25.

In Figure 1, the heating fluid is supplied to heating pipes 27 shown schematically and located within the vessels so that the fluid is not in contact with the materials within the vessel but merely acts to supply heat.

The vessels contain illumination systems including lights 28 located within the vessels. The intention is that the vessels receive wholly generated light without any intention to receive natural light or to transmit that natural light into the growing medium within the vessel. The lights can be LED or fluorescent tubes which are arranged in an array described hereinafter to provide suitable illumination into the liquid medium within the vessels to provide a growing action for the organisms within the medium. In Figure 1, the lights 28 are powered by an electric supply 29 taken from the site electricity supply. Thus there is no intention to utilize the power or heat from the engine 10 to generate power locally to generate the power for the lights 28. It has been found that the system utilizing the site electricity supply is more simple and more suitable without adding the complexity of adding electricity manufacture on site. However this possibility is shown in Figure 6 and described in more detail hereinafter.

In general each vessel contains a bath of a liquid medium, generally water at a required pH. The liquid medium is maintained at the required temperature and at the required pH by carefully monitoring the temperature and pH at various locations within the vessel to ensure that the organisms are maintained within the optimum growing conditions at all places within the vessel.

The liquid medium for each of the vessels is maintained separate so that each vessel has its own liquid and its own liquid processing and operating system. Thus as shown schematically in Figure 1, the liquid for the vessel 21 is injected at an inlet location 30 of the vessel and is extracted at an outlet 31. The liquid is then recirculated back to the inlet through a processing system schematically indicated at 32.

Symmetrically each of the vessels 18, 19 and 20 contains the same processing system schematically indicated at 32. For convenience of illustration the processing system is shown in more detail in respect of vessel 18. Thus the processing system 32 of the vessel 18 includes a duct 33 supplying the extracted liquid containing the organisms at their end of their process within the vessel so that the liquid is carried to an algae separation system schematically indicated at 34.

In general the vessel is arranged so that the passage of the liquid medium through the vessel carries out a growing action on the organism so that the percentage of the algae within the liquid medium increases from the input 30 at a value of the order of 3% through to the outlet 31 where the value is of the order of 6%. As described in more detail hereinafter, the density or concentration of

It will be appreciated that these numbers are only typical and that the numbers may vary dramatically. However the intention is that the algae be introduced to vessel at a lower level with the ability to reproduce within the vessel to a level which is generally the maximum that can be obtained within the illumination system so that the difference between the maximum and minimum levels can be extracted by the algae separation systems 34, returning the minimum level to the vessel for a further passage through the vessel in a further cycle. It will be appreciated that the system is continuous in that the medium contained minimum level of algae is introduced continuously into the vessel and is extracted continuously at the outlet so that the content of algae gradually increases as the medium passes through the vessel in a in a path to the outlet.

Various techniques for separation of the algae are well known and can be used from commercial systems generally including centrifuge systems which in effect increase the concentration of the algae within the liquid medium and extract the portion of increased concentration leaving the remaining portion to be returned. Thus the extracted materials contains a proportion of the liquid medium which is then extracted for return to the system or is expelled in a dewatering system generally indicated at 35. The dewatering system 35 can use heat from the exchanger 22 carried along a line 36 to a dewatering system. Suitable dewatering systems are commercially available.

From the dewatering system the extracted algae and concentration are supplied to a processor 37 again which is of a conventional nature which is used to process the algae to produce a fuel in a fuel supply 38. Generally the algae selected produces bio-diesel.

From the algae separation system 34, the extracted liquid medium containing the minimum amount of algae is returned to the inlet 30 but is passed through a processing step indicated at 39 where the inlet materials are analyzed and the necessary additives supplied to the materials to ensure that the liquid medium contains the required levels of algae and required levels of further materials when introduced at the inlet 30. For this reason there is provided an algae supply 40 and a nutrient supply 41 which supplies the required materials to the step 39. Thus when the material is added into the vessel they are at the required levels for nutrient, pH and algae content.

The exhaust gas on the line 23 is passed through each of the vessels in turn so that they are arranged in series relative to the gas supply. Thus the supply line 23 supplies a gas inlet 42 which enters the vessel 21. After processing within the vessel 21 the gas is extracted at an outlet 43 which is then transferred to the inlet of the vessel through a pump 43A. Thus the gas passes, in the arrangement of Figure 1, through each of the vessels in turn thus emerging from the vessel 18 finally after the final processing step where the gas is supplied to a discharge duct 44 where the gas is released to atmosphere at 45. It will be appreciated therefore that the amount of carbon dioxide within the gas decreases as it enters and passes through each vessel.

The parameters within the vessels are therefore selected independently of one another so as to maximize the processing of the materials within the respective vessel. It will be appreciated therefore that the amount of CO2 within the last vessel 18 is significantly reduced relative to the first vessel 21. For this reason the dwell time of the liquid and the algae within the vessel 18 must be significantly greater than the dwell time within the vessel 21. For this reason the flow rate through the vessel can be significantly higher in the vessel 21 than in the vessel 18. Alternatively the vessels may be of different sizes. In each case the intention is to ensure that the growth of the algae is optimized within the vessel so that the algae levels enter at the predetermined minimum level and exit at the required elevated level.

A further processing characteristic which can be adjusted within the individual vessels is that of a recirculation system generally indicated at 46. Thus each of the vessels has a recirculation system for the gas so that the gas is returned into the vessel for recirculation. The proportion which is recirculated can be controlled so that the number of recirculation's is controlled and can be varied to manage the system within the bio-reactor.

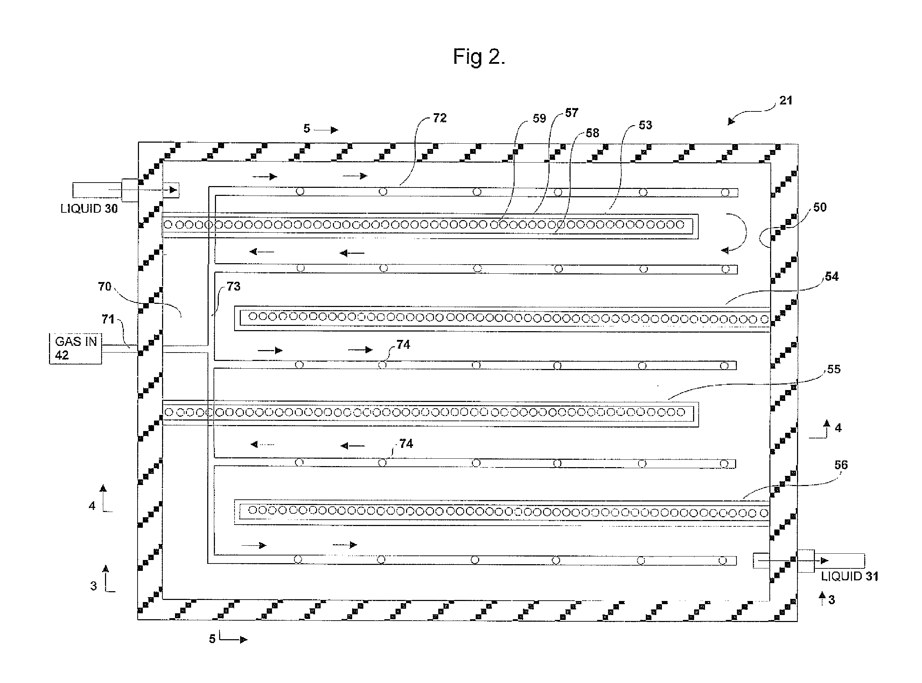

Turning now to Figures 2, 3, 4 and 5, there is shown a respective one of the vessels. As the vessels are identical only one is shown and is indicated generally at 21. The vessel comprises a rectangular container with four side walls 50 upstanding from a base and covered by a top cover 51. Each of the walls and the top cover are insulated by an insulating material 52 so as to maintain heat within the vessel. It is expected that these vessels will be operating in low temperature environments so that there will be a requirement for additional heat rather than cooling.

Inside the outer walls, there are provided a series of divider walls 53, 54, 55 and 56. These are arranged so that the walls 53 and 55 extend from one end wall and are spaced from the other end wall and symmetrically the dividers 54 and 56 extend from the other end wall and extend back toward the first end wall from which they are spaced. This forms a labyrinthine path from the liquid inlet 30 to the liquid outlet 31.

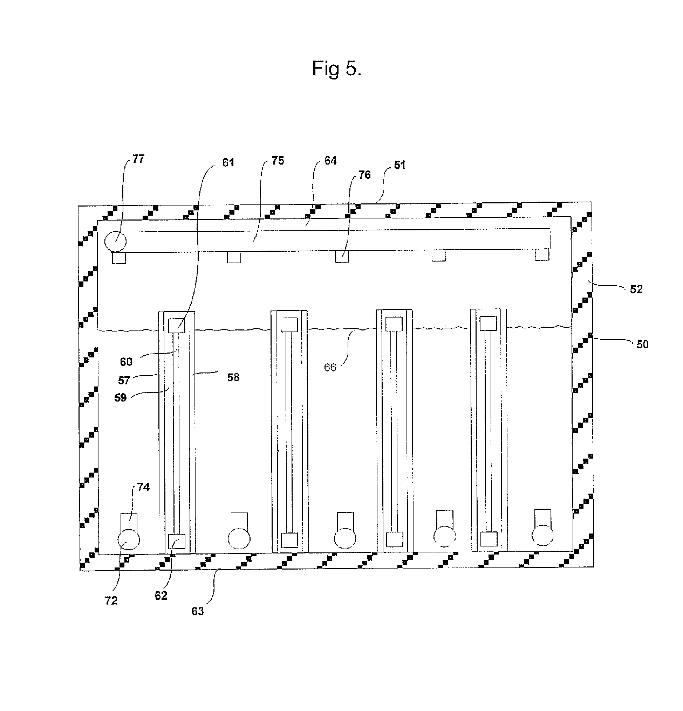

Each divider is formed from a pair of transparent sheets 57 and 58 which form between them a space 59 separated from the liquid within the vessel and flowing through the labyrinth dine path. Inside this space 59 as best shown in Figure 5 is provided a plurality of fluorescent tubes 60 which extend from an upper connector bar 61 to a lower connector bar 62 so that the fluorescent tubes extend through the full height of the bath with the bar 62 closely adjacent the base 63 of the vessel and the bar 61 just below the top of the divider walls. The divider walls terminate at a position spaced from the top panel 51 to leave a header space 64 above the divider walls. The liquid level 66 is maintained of course below the top of the divider walls. Thus the fluorescent tubes 60 are maintained in a liquid free area to avoid interference with their operation.

The path defined inside the vessel is therefore generally rectangular defined by the side walls of the vessel and the divider walls without any complexity of the light tubes and therefore can be readily cleaned by a scraping system which can pass through the path.

The liquid therefore passes from the inlet 30 to the outlet 31 at a rate determined by the rate of flow of the liquid as it is pumped through the path and through the return system including the processor 32.

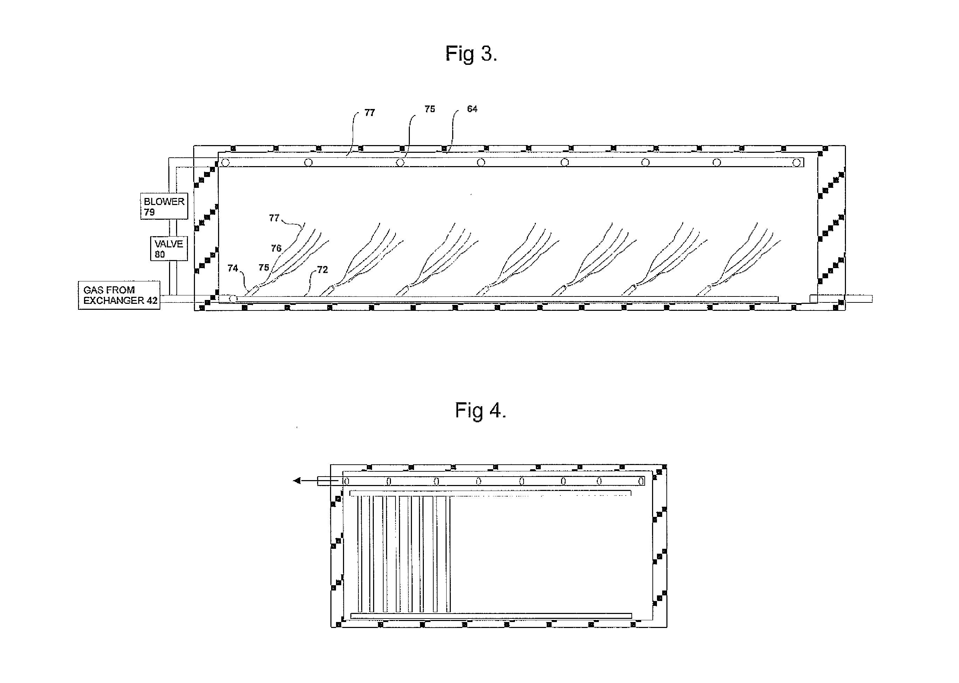

The gas from the exhaust is introduced into the liquid within the vessel through a gas injection system schematically indicated by the gas inlet 42. This includes a duct system 70 including a pipe 71 supplying the gas into the interior of the vessel and a series of pipes 72 extending from that inlet pipe 71 as branches from a header pipe 73. Each of the pipe 72 extends therefore along the path portion defined between the outside wall and the first divider wall or between the divider walls themselves. In the arrangement shown where there are four divider walls, there are therefore five individual paths and five pipes 72. The pipes 72 are located at the base or under the base as supply ducts. On each pipe is provided an injector 73, 74. The injectors 74 are ceramic diffusers arranged to generate bubbles in the gas as it escapes from the pipe into the liquid. Porous ceramic diffusers thus carry the gases and divide the gases into individual small streams to generate small bubbles within the liquid. As shown best in Figure 3, the directional diffusers 74 are arranged at an angle inclined to the base in a direction along the path portion tending to carry the liquid in the required direction along the path. Thus each inclined diffuser includes an end face 75 where the gases emerge in a stream 76 of bubbles which is inclined along the path and also rises within the liquid as indicated at 77. This acts to generate a stream of small bubbles within the liquid and at the same time acts to cause slight agitation within the liquid and slight flow of the liquid along the path. The amount of injection is maintained so that the turbulence within the liquid is below a level which can damage the algae. It will be appreciated that turbulence above certain levels causes a shearing action within the structure of the algae which can break the cells and thus interfere with the proper growth. The provision of the directional diffusers provides a mixing of the bubbles to maximize the dissolving of the carbon dioxide into the liquid and the integration of the carbon dioxide with the algae for the photosynthesis to occur which leads to the growth.

Within the head space 64 at the top of the divider walls is provided a gas extraction system for the recirculation including a plurality of extraction pipes 75 extending across the header space 64 and providing a series of inlet openings 76 along those pipes. The pipes connect at one end to a common discharge pipe 77 carrying the extracted gas to an exterior transfer pipe 78 to a blower 79 and a valve 80 which controls the amount of gas extracted through the recirculation system. The gas in the recirculation system is returned to the inlet 42 which is then re-injected through the system 70.

The gas outlet 43 can be provided as part of the recirculation system or can be provided by a separate gas extraction pipe which draws gas from the vessel at a rate equal to the gas inlet supply rate.

Thus the gas is transported through each of the vessels in turn and the carbon dioxide is extracted to be replaced using the photosynthesis process with oxygen which is expelled into the exhaust gases primarily nitrogen, for discharge eventually at the atmosphere discharge 45.

The CO2 content of the gas at the inlet 42 and at the outlet 43 is measured by a sensor 42A, 43A for managing the process within the respective vessel. The CO2 content can be used to control various parameters including particularly the volume recirculated through the respective vessel.

Turning now to the arrangement shown in Figures 6 to 10, this is basically of the same construction described above so that common elements are identified by the same reference numbers.

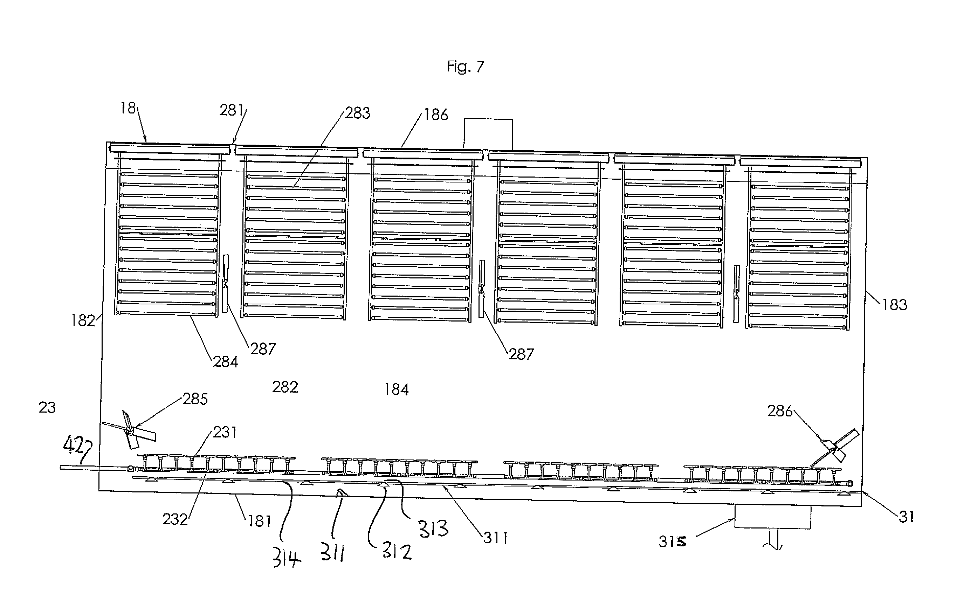

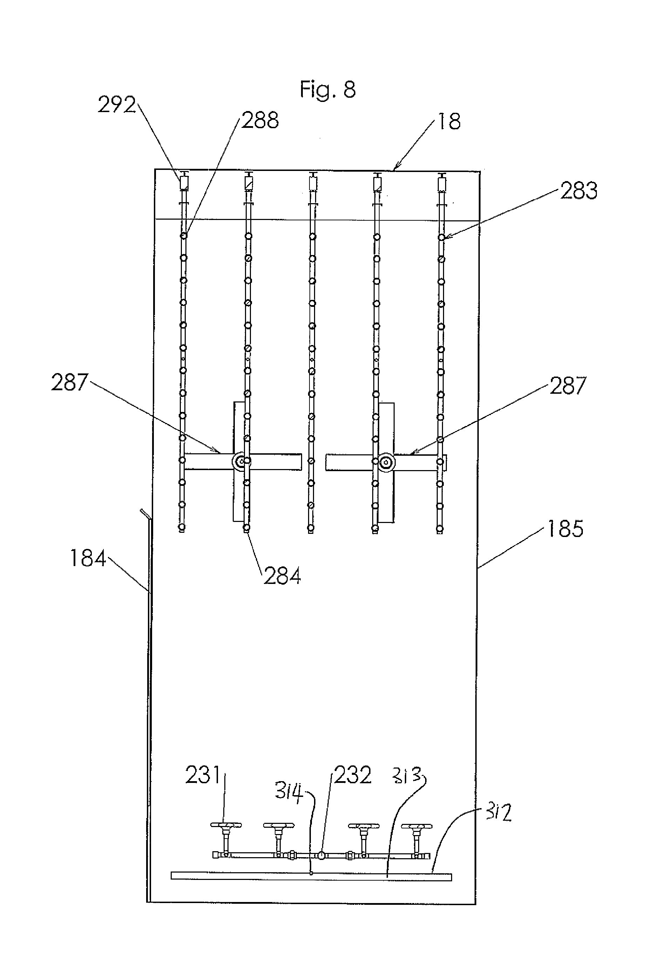

As best shown in Figures 7 and 8, the vessel 18 defining the bioreactor cell is single vessel defined by a base 181, upstanding end walls 182 and 183 and upstanding side walls 184 and 185 forming a rectangular container of simple construction. There is therefore merely a common hollow interior with no pathways of separate chambers.

It will be noted that the system operates to directly apply the exhaust gases to the liquid medium after cooling to a temperature which avoids overheating the liquid. This is possible particularly in view of the use of natural gas as a fossil fuel, bearing in mind that combustion of natural gas generates much lower levels of toxic materials than do other fuels. Thus the application of the un-processed natural gas exhaust into contact with the organisms does not cause toxic effects on the organisms allowing them to flourish in the medium to process the carbon dioxide in the gas.

In this embodiment, rather than using power from the external electricity supply to supply power for the lights to generate the required light energy or illumination to fuel the growth, an electricity generation system 221 in the form preferably of an organic Rankin cycle engine is provided. This receives heat from the exhaust via a branch of the heat exchanger 22 to generate electric power which is then used to drive the system. In particular power can be supplied to the pump 43A, the blower 16, a pump 30A driving the water supply at the inlet 30, the lighting banks 28 and the separation system 34. In this way the system can be self sufficient for energy using only the excess heat from the engine 10.

The system shown in Figure 6 to 10 therefore provides a method for extraction of carbon dioxide from exhaust gases from combustion of a fossil fuel where the fuel is preferably used in an engine using natural gas as the supply. The method includes providing a supply 11 of exhaust gas from combustion of a fossil fuel in the engine 10. Heat is extracted from the exhaust gas using the heat exchanger 22 to cool the exhaust gas and to generate the supply 24 of a heated liquid. In Figure 6, water vapor is extracted at 222 from the exhaust gas in the heat exchanger or at a specifically defined extraction system to form a supply of water.

The vessel 18 contains the liquid medium including or primarily water which carries the photo-synthetic organisms. Light energy is provided by the lighting banks 28 and the cooled exhaust gas are supplied to the liquid medium in the vessel to carry out the growth of the organisms using photosynthesis. The organisms are extracted from the liquid medium by the extraction system 31 allowing harvesting at least some of the organisms from the extracted liquid medium at the separation system 34 and return of some of the liquid medium including some of the water after the extraction to the vessel 18.

In the arrangement of Figure 6, at least some of the supply of water 222 from the heat exchanger is supplied to the vessel via a pipe and the pump 30A to provide a make-up supply of water to replace water removed during the extracting of the organisms or by other losses.

The amount of water which is available to be extracted from the exhaust gas is greater than the amount of water removed during the extracting of the organisms so that the system is water supply positive. This is a particular advantage where water is not available or is in short supply.

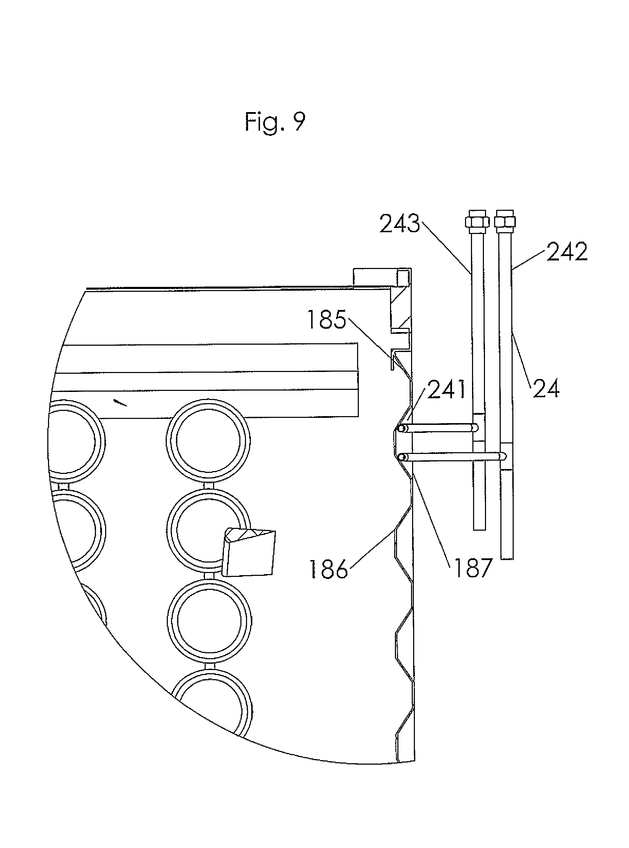

As shown best in Figure 9, the heating of the wall 185 of the tank 18 using the heated liquid from the heat exchanger 22 is effected by passing the liquid through at least one heating duct 241 mounted in contact with at least one wall 185 of the vessel so as to apply heat to the wall 185. More than one duct can be provided ion the wall. Only one or more than one of the walls may be heated depending on the temperature required and the temperature of the liquid, bearing in mind the necessity to maintain the required temperature of the medium without overheating.

The duct 241 is carried on an outside surface of the wall so that it does not interfere with maintaining the inside surface of the wall flush for cleaning. In the example shown the walls are corrugated as indicated at 186 leaving convenient hollows on the outside which can be closed by a cover 187 to define the duct to be heated. The duct can be filled with a suitable heat transfer liquid such as glycol to receive the heat from the hot supply pipes 242 before returning the heating liquid through the pipes 243 to the heat exchanger. The use of the wall as a heating surface avoids introducing heating coils into the medium which can interfere with flow and can require cleaning.

As shown in Figure 7, the cooled exhaust gas from the pipe 23 is supplied to the vessel 18 though the pump 43A and the pipe 42 to a plurality of diffusers 231 at the base of the vessel. The diffusers comprise commercially available circular ceramic disks into which the gas is introduced to release the gas into the medium as a stream of bubbles. The number and dimensions of the diffusers can vary according to engineering practice. The total area of the diffusers combined is preferably of the order of 0.00005 m2 per liter of liquid medium (0.00204 ft2 per US Gallon of liquid medium)

The gas is supplied to the diffusers through a plurality of communication conduits 232 passing through the medium in the vessel and connected to the pipe 42 at one end of the vessel. The conduits form an array of pipes located in the medium at the bottom of the vessel and include heat transfer fins or the like to assist in transferring heat from the gas to the medium. Thus the communication conduits or pipe array 232 is arranged to act as a heat exchanger transferring heat from the gas to the medium in the vessel. This heat transfer allows the gas to enter the vessel at a higher temperature than would otherwise be possible since the gas is further cooled before it is released into the medium through the diffusers. This reduces the heat transfer capacity required for the heat exchanger 22 and improves heat transfer efficiency. The use of the heating through the tank wall and the heat exchange at the bottom of the vessel by the supply pipes ensures effective heat transfer to keep the medium at the required temperatures even in the coldest climate while avoiding hot spots or overheating of the medium. Thus the heat supplied by the cooled exhaust gas and by the heating liquid is controlled to maintain the temperature of the medium in the vessel at a temperature for active growth of the organisms. The supply construction 232 is defined by a horizontal array of supply pipes extending across the base of the vessel.

For recirculation of the liquid medium, the liquid medium is extracted by a suction system 311 extending across the base underneath the supply construction and connected to the duct 31. The suction system includes an array of outlet openings so that the extraction occurs across the whole area of the base to ensure continual extraction of any materials such as particulates collecting at the base. The suction system comprises a plurality of transversely extending ducts 312 at spaced positions along the length of the vessel and each defining an upwardly facing slot 313 across the base. The transverse ducts are connected to a longitudinal pipe 314 along the center of the vessel. For occasional cleaning at a shut down, there is provided a sump 315 in the base 181.

It is well known that organisms such as algae require a light cycle to simulate day and night for proper growth. In such simple organisms the period of light and dark does not need to be controlled to a 24 hour clock but there need to be periods where the organism is subject to dark and periods of illumination for growth. In the absence of such cycling the organisms do not function properly to cause the required level of photosynthesis to sustain life while avoiding excess use of the available chlorophyll.

Typically such light and dark cycles are created by the simple expedient of turning the illumination source on and off at suitable periods which can be measured in hours or parts of seconds.

In the present arrangement the required light and dark cycles are generated by forming a lighted area 281 in the vessel in which the medium is supplied with light energy and a dark area 282 in the vessel in which the medium is remote from the light energy. The medium containing the organisms is then circulated between the light and dark areas to generate the light and dark cycles for the organisms. The lighted area is formed by a series of light banks 283 in the vessel extending from the top 186 to a bottom 284 of the light banks spaced from the base 181. As is well appreciated, the density of the organisms in the medium is such that the light can only penetrate from a source such as a florescent tube to a depth of the order of a few inches at most. Thus as soon as the organisms move beyond this distance from the light source they are in the dark area.

The ratio of light area to total area in the vessel is typically in the range 50 to 80% which simulates the level of light which the organism might meet in normal existence in the day to night light cycle, thus maximizing the growth potential while avoiding the over stimulation of the organisms which interferes with growth..

In this arrangement, the lighted and dark areas are open to one another at the bottom of the light banks within the vessel. Thus the interface between the lighted and dark areas is formed by an end face of the banks of light sources without any impediment to flow therebetween. The circulation between the areas is caused by free currents within the vessel generated by circulation fans 285 and 286 located suitable within the vessel. In the example shown the fans are located at the bottom corners within the dark area of the vessel to generate complex flow and turbulence within the vessel. These fans are supplemented by additional fans between one bank 283 and the next and tending to carry the medium though the banks. Thus the circulation is effected without a pathway or guide which defines a specific path of the medium and the medium is free to move within the vessel.

In the lighted area, the light sources are arranged at spacing to provide light to substantially all of the organisms in the lighted area. In the dark area all the organisms are dark. It will be appreciated that, in the absence of any guide to accurately control the flow of the medium through the light and dark areas, the individual organisms may see very different ratios of light and dark even though the average will see a ratio of light and dark equal to the ratio of volume in the light and dark areas. However the organisms are themselves are not average or homogenous and vary in their characteristics and requirements depending on their stage of life. Thus it has been found that the random ratios which are generated by the random or uncontrolled flow of the medium in the light and dark areas actually leads to a better growth rate than does a more specific or accurate control; over the light /dark cycle. The rate of circulation can be varied to maximize the growth rate of the organisms either by changing the flow patterns by redirecting the circulation fans of by increasing the number or flow rate of the fans.

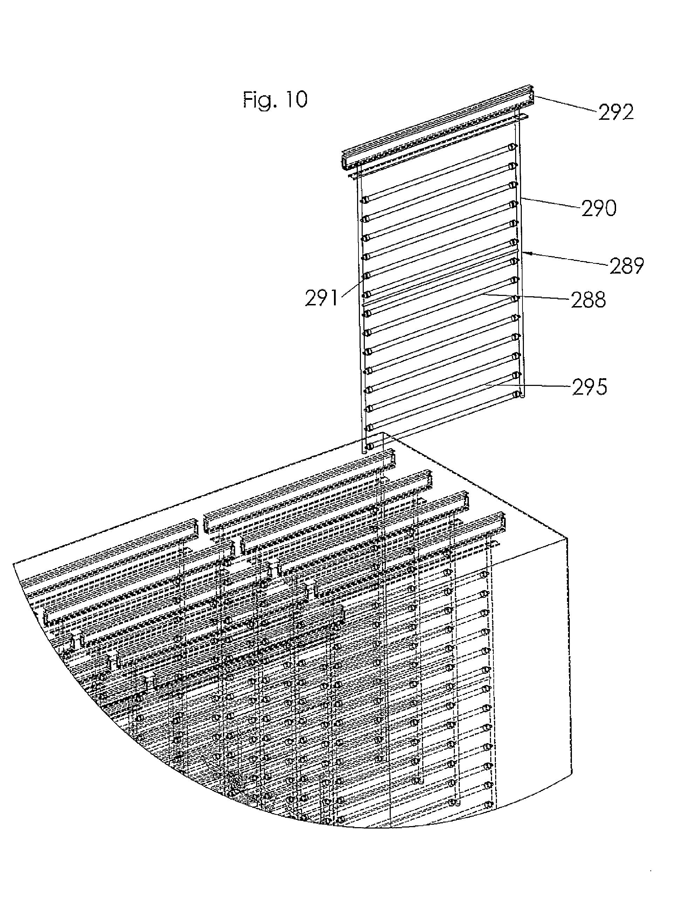

As shown in Figure 10, the light energy is supplied by the plurality of banks 283 of light sources which are suspended into the vessel from supports at the top. In the example shown there are six banks at spaced positions along the vessel. Each bank comprise a series of rows side by side across the vessel. Each row includes a plurality of parallel spaced florescent tubes 288 carried on a common mounting assembly 289 defined by vertical side rails 290 and 291. A top housing 292 contains the electrical components and supports the rails. The row is dropped into the vessel from the top and sits on a pair of wires 293 across the opening so that the wires support all the rows of the bank. Each florescent tube is surrounded by a protecting sleeve 295 which allows cleaning of the light system to ensure continued light penetration. Thus the tubes of each row are horizontal and the rows are vertical and arranged side by side across the vessel with each row extending longitudinally of the vessel.

It will be appreciated that, as shown in Figure 10, the banks of light sources can be removed from the vessel through the top. This leaves the internal surface of the walls free from any mounting for the lighting system.

Similarly the supply construction 232 and the diffusers 231 can be removed through the top by disconnecting the end of the array from the pipe 42. When removed in this way, the upstanding side walls are flush with no outstanding components allowing cleaning of the upstanding flush side walls.

Similarly the suction system 311 including the ducts 312 and the pipe 313 can be removed through the top by disconnecting the end of the array from the pipe. When removed in this way, the upstanding side walls and base are flush with no outstanding components allowing cleaning of the upstanding flush side walls.

The use of exhaust direct from the natural gas engine, the use of high levels of light energy and the recirculation of the medium and the gas as described herein allows the organisms to be grown to a density of the organisms in the medium greater than 0.5 grams per liter of liquid medium (0.075 ounces per US Gallon of liquid medium). This is many times greater than is typically obtained with systems of this type. Typically the arrangement described above can generate a density greater than the value of 0.5 grams per liter and commonly in the range 0.9 to 1.8 grams per liter (0.12 to 0.24 ounces per US Gallon of liquid medium).

In order to achieve this:
the level of carbon dioxide introduced into the medium is in the range 1.062 x 10-7 to 4.249 x 10-7 m3/sec per liter of liquid medium (0.0008517 to 0.0034 CFM per gallon of liquid medium),
the recirculation system is arranged so that the gas has a retention time of greater than 0.5 to 3.0 seconds per foot of liquid medium depth (1.64 to 9.84 seconds per meter of liquid medium depth),
the level of light energy introduced into the medium is greater than 0.045 to 0.45 watts per liter (or 0.170 to 1.7 watts per US gallon) of liquid medium
and the rate of extraction is 1.5 to 4.5 grams per liter of liquid medium per day (0.20 to 0.6 ounces per US Gallon of liquid medium per day).

Since various modifications can be made in my invention as herein above described, and many apparently widely different embodiments of same made within the spirit and scope of the claims without department from such spirit and scope, it is intended that all matter contained in the accompanying specification shall be interpreted as illustrative only and not in a limiting sense.

## Claims

1. A method for extraction of carbon dioxide from exhaust gases from combustion of a fossil fuel, the method comprising:
providing a supply of exhaust gas from combustion of a fossil fuel;
extracting heat using a heat exchanger to cool the exhaust gas and to generate a supply of a heated liquid;
extracting water vapor from the exhaust gas to form a supply of water;
providing a vessel containing a liquid medium including water carrying photo-synthetic organisms;
providing light energy to the liquid medium;
supplying the cooled exhaust gas to the liquid medium in the vessel;
extracting the liquid medium containing the organisms from the vessel, harvesting at least some of the organisms from the extracted liquid medium and returning some of the liquid medium including some of the water after the extraction to the vessel;
and adding at least some of the supply of water to the vessel to provide a make-up supply of water to replace water removed during the extracting of the organisms.

2. The method according to claim 1 wherein the amount of water extracted from the exhaust gas is greater than the amount of water removed during the extracting of the organisms.

3. The method according to claim 1 including heating the vessel using the heated liquid by passing the liquid through at least one heating duct mounted in contact within with an outside surface of at least one wall of the vessel so as to apply heat to the wall.

4. The method according to any one of claims 1 to 3 wherein the inside surface of the wall is flush for cleaning.

5. The method according to any one of claims 1 to 4 wherein the cooled exhaust gas is supplied to the vessel though a plurality of diffusers at the base of the vessel supplied through a plurality of communication conduits passing through the medium in the vessel and wherein the communication conduits are arranged to act as heat exchanger transferring heat from the conduits to the medium in the vessel.

6. The method according to any one of claims 1 to 5 wherein there is provided a heat transfer system arranged to use heat from the heat exchanger to dewater the liquid medium from the liquid medium during the extracting of the organisms.

7. The method according to any one of claims 1 to 6 wherein the vessel has a respective liquid medium extraction system arranged to continuously extract the liquid medium containing the organisms from said the vessel and a harvesting system arranged to extract some of the organisms from the extracted liquid medium so as to provide a stream of harvested organisms in a liquid medium and a stream of liquid medium to be returned to the vessel.

8. The method according to any one of claims 1 to 7 wherein there is provided a gas recirculation system for withdrawing gas from the vessel and returning the gas to the gas supply system to the liquid medium in the vessel.

9. The method according to any one of claims 1 to 8 wherein the engine is an engine fueled by natural gas and used for natural gas compression.

10. The method according to any one of claims 1 to 9 wherein the light energy is supplied to the vessel so as to generate a lighted area in the vessel in which the medium is supplied with light energy and to generate a dark area in the vessel in which the medium is remote from the light energy and wherein light and dark cycles for the organisms are generated by circulating the medium between the light and dark areas.

11. The method according to any one of claims 1 to 10 wherein the light energy is supplied by a plurality of banks of light sources, wherein the gas is supplied to the vessel by a supply construction defined by a plurality of diffusers at a base of the vessel and supply pipes extending across the base and including the steps of removing the banks of light sources from the vessel through the top, removing the supply construction and, when the light sources and the supply construction are removed the upstanding side walls are flush with no outstanding components, cleaning the upstanding flush side walls.

12. The method according to any one of claims 1 to 11 wherein the density of the organisms in the medium is greater than greater than 0.5 grams per liter of liquid medium (0.075 ounces per US Gallon of liquid medium).

13. The method according to any one of claims 1 to 12 wherein the level of carbon dioxide introduced into the medium is greater than .062 x 10-7 m3/sec per liter of liquid medium (0.0008517 CFM per gallon of liquid medium).

14. The apparatus according to any one of claims 1 to 13 there is provided a gas recirculation system for withdrawing gas from the vessel and returning the gas to the gas supply system to the liquid medium in said at least one vessel and wherein the recirculation system is arranged so that the gas has a retention time of greater than 0.5 seconds per foot of liquid medium depth (1.64 seconds per meter of liquid medium depth) and wherein the rate of extraction is greater than 1.5 grams per liter of liquid medium per day (0.20 ounces per US Gallon of liquid medium per day).

15. The method according to any one of claims 1 to 14 wherein the level of light energy introduced into the medium is greater than 0.045 watts per liter (or 0.170 watts per US gallon).
